# EUROPEAN PATENT SPECIFICATION

(11) **EP 2 949 358 B1**
(45) Date of publication and mention of the grant of the patent: **31.05.2017**
(21) Application number: 15163480.5
(22) Date of filing: 14.04.2015
(51) Int. Cl.: A61M 25/00

(54) **CATHETER**
KATHETER
CATHÉTER

(30) Priority: 21.05.2014 JP 2014104918
(43) Date of publication of application: 02.12.2015
(73) Proprietor: ASAHI INTECC CO., LTD., Nagoya-shi, Aichi 463-0024 (JP)
(72) Inventor: Ishikawa, Masatomo c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 2 174 685
- JP-A- 2007 236 472
- US-A1- 2009 281 610
- US-A1- 2011 245 775

## Description

### BACKGROUND OF THE INVENTION

### 1. Field of the Invention

The present invention relates to a catheter inserted in a blood vessel, etc. for use.

### 2. Description of the Related Art

There is known a catheter inserted in a lumen of a blood vessel, etc. for use. With the use of a catheter, a physician injects liquid chemicals or a contrast agent into a blood vessel or sends various devices (a stent or an embolic coil, for example) to a lesion of a blood vessel.

The catheter is constituted by a resin tube and various reinforcing bodies reinforcing the tube. For example, European Unexamined Patent Application No. 1120127 describes that a coil body formed by winding a plurality of single-strand wires into a helical coil structure is used as a reinforcing body of a catheter. Moreover, there is proposed a technique of improving rotation transmission performance and press-in performance (pushability) of a catheter by using, as some of a plurality of single-strand wires constituting the coil body, thicker element wires (thick wires) than other element wires (thin wires) (U.S. Unexamined Patent Application No. 2007/0208368, for example).

However, the conventional catheter described above has a problem of unevenness occurring on an outer peripheral surface or an inner peripheral surface of the coil body because the thin wires and the thick wires constitute the coil body. This consequently causes a problem of increasing sliding resistance between the outer peripheral surface of the catheter and an inner wall of a blood vessel or a lesion, for example, or increasing sliding resistance between the inner peripheral surface of the catheter and a combined device such as a guide wire. Moreover, the occurrence of unevenness on the outer peripheral surface (or the inner peripheral surface) of the catheter makes it difficult, when covering the outer peripheral surface (or the inner peripheral surface) of the catheter with coating, to sufficiently adhere the coating. EP2174685 discloses a catheter comprising a tube body including an inner layer, a reinforcing layer covering the inner layer, and an outer layer covering the reinforcing layer, wherein the reinforcing layer is a coil body formed by winding a plurality of single-strand wires comprising thin wires and thick wires into a helical coil structure.

### SUMMARY OF THE INVENTION

In view of the above problems of the conventional technique, the present invention aims at preventing occurrence of unevenness on an outer peripheral surface or an inner peripheral surface of a catheter including a coil body constituted by large-diameter or thick wires and small-diameter or thin wires.

In order to solve the problems described above, the catheter of the invention adopts the following structure as defined in claim 1. That is, the catheter includes a tube body including an inner layer, a reinforcing layer covering the inner layer, and an outer layer covering the reinforcing layer. The tube body may form a catheter shaft extending from a proximal end to a distal end. The reinforcing layer is a coil body formed by winding a plurality of single-strand wires comprising small-diameter or thin wires and large-diameter or thick wires into a helical coil structure. The coil body includes a length part in which the thick wires are ground so that the cross-sectional shape of the thick wires is formed to have a substantially semicircular shape, i.e. to have the shape of a circular segment, and an inner diameter and an outer diameter of the coil body are constant in the part in which the thick wires are ground.

Such a catheter of the invention includes a length part in which the thick wires are ground so that the cross-sectional shape thereof is formed to have a substantially semicircular shape, and an inner diameter and an outer diameter of the coil body in this part are constant. Thus, no unevenness occurs on the outer surface (or the inner surface) of the coil body. Therefore, it is possible to suppress sliding resistance on the surfaces of the catheter and secure adhesion of coating.

Moreover, as described in detail later, in the catheter of the invention, when the catheter is bent, the thin wires move along the circular arc portion of the thick wires, which allows the catheter to bend largely. As a result, it is possible to exert favorable following properties even for a largely bent blood vessel.

Moreover, in the catheter of the invention described above, a straight portion of the substantially semicircular shape of the thick wires may be disposed on the inner side of the coil body or on the outer side of the coil body.

In such a catheter of the invention, if the straight portion of the section (substantially semicircular shape) of the thick wires is disposed on the outer side of the coil body, it is possible to easily produce the catheter of the invention only by forming the coil body by wiring thick wires and thin wires and then grinding the thick wires from the outer side of the coil body.

Moreover, in the catheter of the invention described above, the coil body has a proximal end and a distal end, wherein the length part in which the thick wires are ground may be provided at a distal end portion of the coil body, and another length part of the coil body, in which the thick wires are not ground may be provided at a proximal end portion of the coil body. In the length part in which the thick wires are not ground, the thick wires may project on the outer side of the coil body.

In such a catheter of the invention, the length part in which the thick wires are ground (a length part with a smooth surface) is provided at the distal end portion of the coil body, and the other length part in which the thick wires are not ground and project on the outer peripheral side (a length part with an uneven surface) is provided at the proximal end portion of the coil body. Therefore, it is possible to easily fix the catheter at a desired position by suppressing sliding resistance at the distal end portion of the catheter particularly requiring sliding properties and moderately securing sliding resistance at the proximal end of the catheter, in addition to the effects of the invention described above.

Moreover, in the catheter of the invention described above, a braid layer may be disposed between the inner layer and the coil body to increase a rigidity of the tube body.

### BRIEF DESCRIPTION OF THE DRAWINGS

FIG. 1 is an explanatory diagram illustrating a structure of a catheter according to a first embodiment of the invention;
FIG. 2 is an explanatory diagram illustrating the detail of a catheter shaft of the catheter according to the first embodiment of the invention;
FIG. 3 is an explanatory diagram illustrating a situation when a coil body of the catheter according to the first embodiment of the invention is bent;
FIG. 4 is an explanatory diagram illustrating the detail of a catheter shaft of a catheter according to a second embodiment of the invention;
FIG. 5 is an explanatory diagram illustrating the detail of a catheter shaft of a catheter according to a third embodiment of the invention; and
FIG. 6 is an explanatory diagram illustrating the detail of a catheter shaft of a catheter according to a fourth embodiment of the invention.

### DETAILED DESCRIPTION OF THE PREFERRED EMBODIMENTS

### A. First embodiment:

In the following, various embodiments of the catheter of the invention will be described so as to clarify the subject matter of the above-described invention.

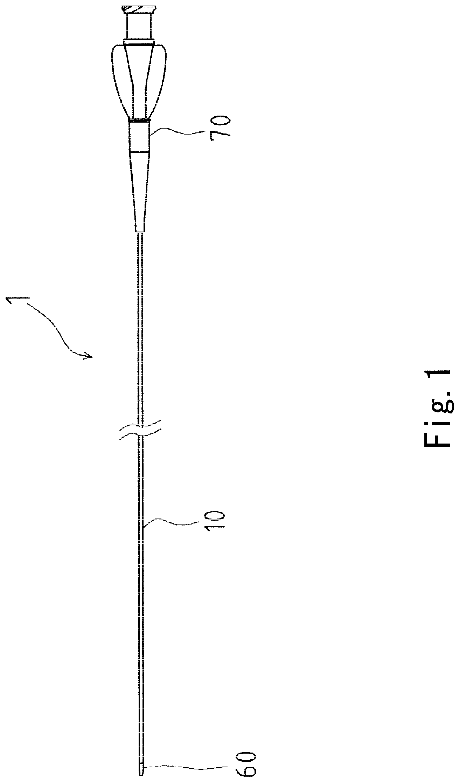

FIG. 1 is an explanatory diagram illustrating a structure of a catheter 1 according to a first embodiment of the invention. As illustrated in FIG. 1, the catheter 1 of the first embodiment is constituted by a catheter shaft 10, a tip 60 provided at the distal end of the catheter shaft 10, and a connector 70 provided at the proximal end of the catheter shaft 10, for example.

Note that the "catheter shaft" in the specification corresponds to the "tube body" of the invention.

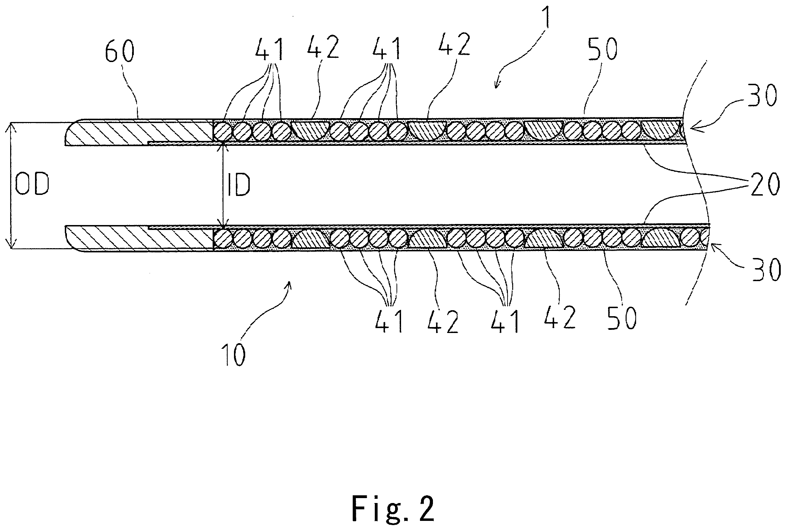

FIG. 2 is an explanatory diagram illustrating the detail of the catheter shaft 10 of the catheter 1 according to the first embodiment of the invention. As illustrated in FIG. 2, the catheter shaft 10 of the first embodiment is a tubular structural body, and is constituted by an inner layer 20, a coil body 30 as a reinforcing layer covering the inner layer 20, and an outer layer 50 covering the coil body 30, for example.

The inner layer 20 is formed of resin. The resin material forming the inner layer 20 is not especially limited. However, considering slidability with an inserted instrument (a guide wire or a catheter, for example), polytetrafluoroethylene (PTFE) is preferable.

The outer layer 50 is also formed of resin. The resin material forming the outer layer 50 is not especially limited, and there can be used polyamide, polyamide elastomer, polyester, and polyurethane, for example.

The coil body 30 is formed by winding two kinds of single-strand wires having different wire diameters (small-diameter or thin wires 41, large-diameter or thick wires 42) into a helical coil structure.

Note that the material of single-strand wires constituting the coil body 30 is not especially limited, and there can be used stainless steel (SUS304, SUS316, etc.), gold, white gold, tungsten, platinum, nickel, and an alloy of such elements, for example.

The resin tip 60 is provided at the distal end of the catheter shaft 10. The resin forming the tip 60 is not especially limited. However, polyurethane, polyurethane elastomer, etc. are used preferably. Moreover, the tip 60 may contain radiopaque powder (tungsten powder, for example). This enables a technician such as a physician to accurately grasp a position of the catheter in coronary angiography.

Here, in the catheter 1 of the first embodiment, the thick wires 42 of the coil body 30 are ground so that the cross-sectional shape there is is formed to have a substantially semicircular shape. Then, the grinding of the thick wires 42 makes the inner diameter ID and the outer diameter OD of the coil body 30 constant.

In such a catheter 1 of the first embodiment, the thick wires 42 are ground so that the cross-sectional shape thereof is formed to have a substantially semicircular shape, i.e. to have the shape of a circular segment, whereby the inner diameter ID and the outer diameter OD of the coil body 30 become constant, and no unevenness occurs on the outer surface (or the inner surface) of the coil body 30. Therefore, it is possible to prevent increase of sliding resistance between the outer surface of the catheter 1 and an inner wall of a blood vessel or a lesion, for example, and an increase of sliding resistance between the inner surface of the catheter 1 and a combined device such as a guide wire.

Moreover, no unevenness occurs on the surface of the coil body 30, which makes it possible to sufficiently secure adhesion of coating (the outer layer 50 in the first embodiment) on the coil body 30.

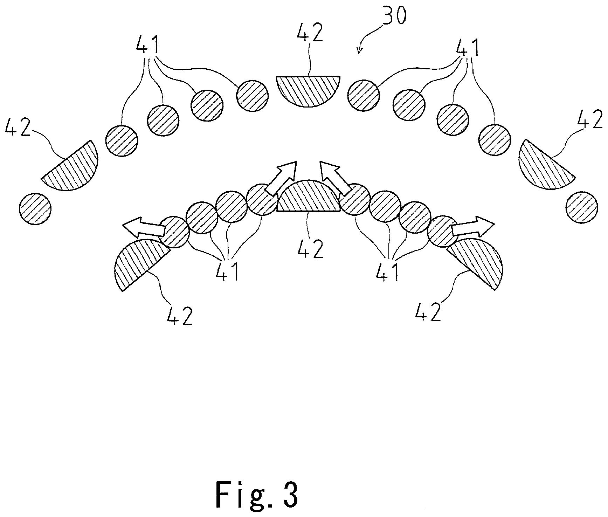

FIG. 3 is an explanatory diagram illustrating a situation when the coil body 30 of the catheter 1 according to the first embodiment of the invention is bent. When the coil body 30 is bent, force is added to the element wires on the bent side (the lower side in FIG. 3) in a direction compressing the coil body 30. Here, as illustrated by white arrows in FIG. 3, the thin wires 41 adjacent to the thick wires 42 slightly move to the lumen side of the coil body 30 as the thin wires 41 are pushed out along the circular arc portion of the thick wires 42.

In this manner, when the coil body 30 is bent, the thin wires 41 move along the circular arc portion of the thick wires 42, which prevents stretching of the coil body 30 and allows the coil body 30 (and then the catheter 1) to bend largely. As a result, it is possible to exert favorable following properties even for a largely bent blood vessel.

### B. Second embodiment:

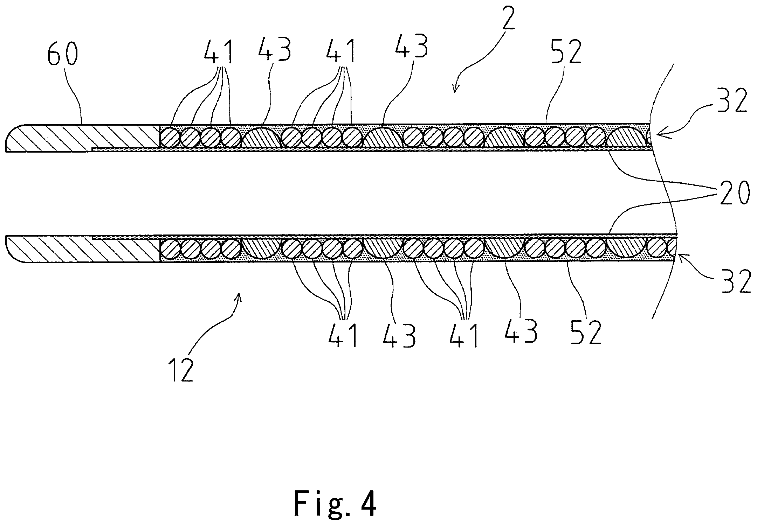

FIG. 4 is an explanatory diagram illustrating the detail of a catheter shaft 12 of a catheter 2 according to a second embodiment of the invention. The illustrated catheter 2 of the second embodiment is different from the catheter 1 of the above-described first embodiment in the following aspect. That is, in the catheter 1 of the first embodiment, the straight portion of the substantially semicircular shape of the thick wires 42 is disposed on the outer side of the coil body 30 (see FIG. 2). By contrast, in the catheter 2 of the second embodiment, the straight portion of the semicircular shape of the thick wires 43 is disposed on the inner side of a coil body 32, as illustrated in FIG. 4.

Regarding aspects other than the above, the catheter 1 of the first embodiment and the catheter 2 of the second embodiment have the same structure. That is, the catheter shaft 12 is constituted by the inner layer 20, the coil body 32 covering the inner layer 20, and an outer layer 52 covering the coil body 32, and the coil body 32 is constituted by the thin wires 41 and the thick wires 43. Moreover, the tip 60 is provided at the distal end of the catheter shaft 12.

Also in such a catheter 2 of the second embodiment, it is possible to prevent occurrence of unevenness on the surface of the coil body 32 (and then the catheter 2), similarly to the catheter 1 of the above-described first embodiment. Moreover, when the coil body 32 is bent, the thin wires 41 move along the circular arc portion of the thick wires 43, which enables the catheter 2 to bend largely.

In fact, the catheter 1 of above-described first embodiment can be produced more easily. That is, this is because, in the catheter 1 of the first embodiment, the straight portion of the substantially semicircular shape of the thick wires 42 is disposed on the outer side of the coil body 30 (see FIG. 2), and thus it is possible to easily produce the coil body 30 only by forming the coil body by wiring the thick wires 42 and the thin wires 41 into a helical coil structure and then grinding the thick wires 42 from the outer side of the coil body.

### C. Third embodiment:

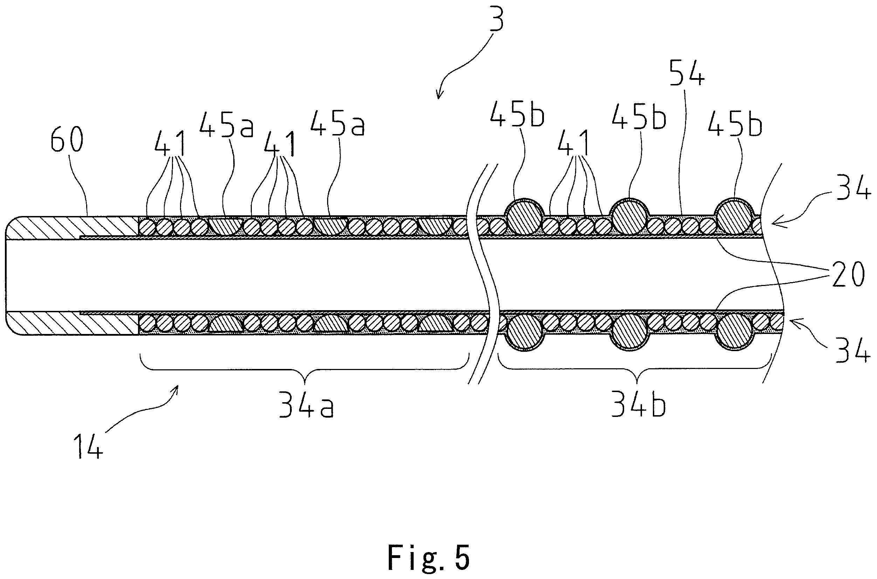

FIG. 5 is an explanatory diagram illustrating the detail of a catheter shaft 14 of a catheter 3 according to a third embodiment of the invention. The illustrated catheter 3 of the third embodiment is different from the catheter 1 of the above-described first embodiment in the following aspect. That is, in the catheter 3, thick wires 45a are ground at a distal end portion 34a, i.e. at a length part, of a coil body 34 so that the cross-sectional shape thereof is formed to have a substantially semicircular shape, while thick wires 45b are not ground at a proximal end portion 34b, i.e. at another length part, of the coil body 34. Then, in the length part in which the thick wires 45b are not ground, the thick wires 45b project on the outer side of the coil body 34, thus forming unevenness on the outer surface.

Regarding aspects other than the above, the catheter 3 of the third embodiment has the same structure as the catheter 1 of the above-described first embodiment. That is, the catheter shaft 14 is constituted by the inner layer 20, the coil body 34 covering the inner layer 20, and an outer layer 54 covering the coil body 34, and the coil body 34 is constituted by the thin wires 41 and the thick wires 45. Moreover, the tip 60 is provided at the distal end of the catheter shaft 14.

Also in such a catheter 3 of the third embodiment, it is possible to prevent occurrence of unevenness on the surface of the distal end portion 34a of the coil body 34 (and then the catheter 3), similarly to the catheters of the above-described various embodiments. Moreover, when the distal end portion 34a of the coil body 34 is bent, the thin wires 41 move along the circular arc portion of the thick wires 45a, which allows the catheter 3 to bend largely. In this manner, the catheter 3 of the third embodiment can exert, at the distal end portion of the catheter 3 inserted in a curved peripheral blood vessel, favorable sliding properties relative to an inner wall of a blood vessel and favorable following properties for a blood vessel.

Meanwhile, in the catheter 3 of the third embodiment, the thick wires 45b are not ground at the proximal end portion of the catheter 3, and thus unevenness is formed on the outer peripheral surface of the coil body 34. This moderately secures sliding resistance at the proximal end portion of the catheter 3, which makes it possible to easily fix the catheter 3 inserted in a lumen of a blood vessel, etc. at a desired position.

### D. Fourth embodiment:

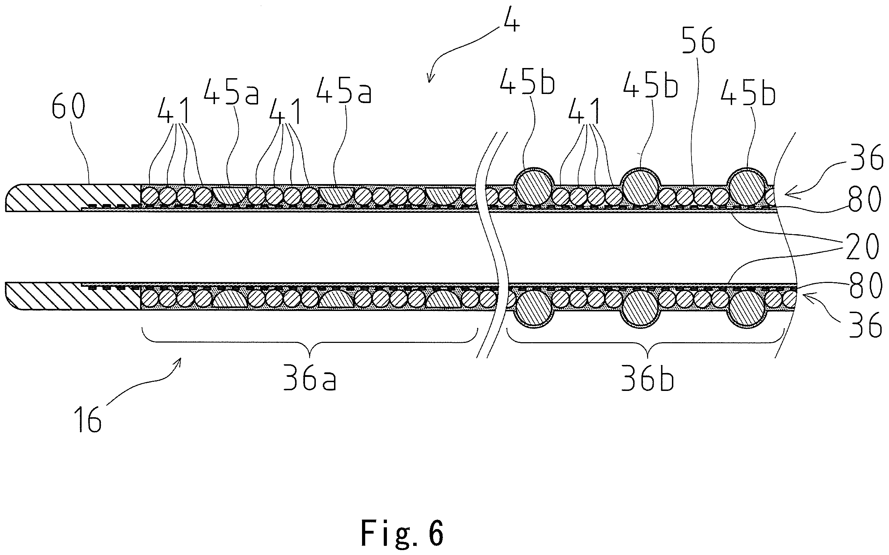

FIG. 6 is an explanatory diagram illustrating the detail of a catheter shaft 16 of a catheter 4 according to a fourth embodiment of the invention. In the catheter 4 of the fourth embodiment, the inner layer 20 is covered by a braid layer 80 formed by braiding metal wires, and the braid layer 80 is covered by a coil body 36. Then, the coil body 36 is covered by an outer layer 56.

Regarding aspects other than the above, the catheter 4 of the fourth embodiment has the same structure as the catheter 3 of the above-described third embodiment. That is, the coil body 36 is constituted by the thin wires 41 and the thick wires 45, and the thick wires 45 are ground at a distal end portion 36a of the coil body 36 (the thick wires 45a) and are not ground at a proximal end portion 36b of the coil body 36 (the thick wires 45b). Moreover, the tip 60 is provided at the distal end of the catheter shaft 16.

Also in such a catheter 4 of the fourth embodiment, it is possible to prevent occurrence of unevenness on the surface of the distal end portion 36a of the coil body 36 (and then the catheter 4), and allow the distal end portion 36a of the coil body 36 to bend largely, similarly to the catheters of the above-described various embodiments.

Furthermore, in the catheter 4 of the fourth embodiment, the braid layer 80 is provided as a reinforcing layer of the catheter 4 in addition to the coil body 36, which increases the rigidity of the catheter shaft 16. In particular, at the distal end portion 36a of the coil body 36, the rigidity reduced by grinding of the thick wires 45 can be compensated by the braid layer 80. As a result, it is possible to easily transmit push-in force added by a technician in a distal end direction to the tip 60.

## Claims

1. A catheter (1; 2; 3; 4), comprising:
a tube body (10) including an inner layer (20), a reinforcing layer covering the inner layer (20), and an outer layer (50) covering the reinforcing layer, wherein
the reinforcing layer is a coil body (30; 32) formed by winding a plurality of single-strand wires comprising thin wires (41) and thick wires (42; 43; 45a) into a helical coil structure, **characterized in that**
the coil body (30; 32) includes a length part in which the thick wires (42; 43; 45a) are ground so that the cross-sectional shape of the thick wires (42; 43; 45a) is formed to have a substantially semicircular shape, and
an inner diameter (ID) and an outer diameter (OD) of the coil body (30; 32) are constant in the part in which the thick wires (42; 43; 45a) are ground.

2. The catheter (1; 3; 4) according to claim 1, wherein
a straight portion of the substantially semicircular shape of the thick wires (42; 45a) is disposed on an outer side of the coil body (30).

3. The catheter (2) according to claim 1, wherein
a straight portion of the substantially semicircular shape of the thick wires (43) is disposed on an inner side of the coil body (32).

4. The catheter (3; 4) according to any one of claims 1 to 3, wherein
the coil body (34; 36) has a proximal end and a distal end,
the length part in which the thick wires (45a) are ground is at a distal end portion of the coil body (34; 36), and
the coil body (34; 36) further has a length part in which the thick wires (45b) are not ground, at its proximal end portion.

5. The catheter (3; 4) according to claim 4, wherein
the thick wires (45b) project on the outer side of the coil body (34; 36) in the length part in which the thick wires (45b) are not ground.

6. The catheter (4) according to any one of claims 1 to 5, wherein
a braid layer (80) is disposed between the inner layer (20) and the coil body (36).

## Patentansprüche

1. Katheter (1; 2; 3; 4) mit:
einem Röhrenkörper (10), der eine Innenschicht (20), eine die Innenschicht (20) umgebende Versteifungsschicht und eine die Versteifungsschicht umgebende Außenschicht (50) aufweist, wobei
die Versteifungsschicht ein Wicklungskörper (30; 32) ist, der aus einer schraubenförmige Wicklung einer Vielzahl von einsträngigen Drähten geformt ist, die dünne Drähte (41) und dicke Drähte (42; 43; 45a) umfassen, **dadurch gekennzeichnet, dass**
der Wicklungskörper (30; 32) einen Längenabschnitt aufweist, in dem die dicken Drähte (42; 43; 45a) so abgeschliffen sind, dass die dicken Drähte (42; 43; 45a) eine im Wesentlichen halbkreisförmige Querschnittsform haben, und
der Innendurchmesser (ID) und Außendurchmesser (OD) des Wicklungskörpers (30; 32) in dem Abschnitt, in dem die dicken Drähte (42; 43; 45a) abgeschliffen sind, konstant sind.

2. Katheter (1; 3; 4) nach Anspruch 1, wobei
die Sehne der im Wesentlichen halbkreisförmigen Form der dicken Drähte (42; 45a) auf der Außenseite des Wicklungskörpers (30) liegt.

3. Katheter (2) nach Anspruch 1, wobei
die Sehne der im Wesentlichen halbkreisförmigen Form der dicken Drähte (43) auf der Innenseite des Wicklungskörpers (32) liegt.

4. Katheter (3; 4) nach einem der Ansprüche 1 bis 3, wobei
der Wicklungskörper (34; 36) ein proximales Ende und ein distales Ende hat,
der Längenabschnitt, in dem die dicken Drähte (45a) abgeschliffen sind, in dem distalen Endabschnitt des Wicklungskörpers (34; 36) liegt, und
der Wicklungskörper (34; 36) in seinem proximalen Endabschnitt einen Längenabschnitt aufweist, in dem die dicken Drähte (45b) nicht abgeschliffen sind.

5. Katheter (3; 4) nach Anspruch 4, wobei
die dicken Drähte (45b) in dem Längenabschnitt, in dem die dicken Drähte (45b) nicht abgeschliffen sind, auf der Außenseite des Wicklungskörpers (34; 36) hervorragen.

6. Katheter (4) nach einem der Ansprüche 1 bis 5, wobei
zwischen der Innenschicht (20) und dem Wicklungskörper (36) eine Geflechtschicht (80) angeordnet ist.

## Revendications

1. Cathéter (1 ; 2 ; 3 ; 4), comprenant :
un corps de tube (10) comprenant une couche interne (20), une couche de renforcement recouvrant la couche interne (20), et une couche externe (50) recouvrant la couche de renforcement, dans lequel
la couche de renforcement est un corps de bobine (30 ; 32) formé par l'enroulement d'une pluralité de fils à brin unique comprenant des fils minces (41) et des fils épais (42 ; 43 ; 45a) dans une structure de bobine hélicoïdale, **caractérisé en ce que**
le corps de bobine (30 ; 32) comprend une partie longueur dans laquelle les fils épais (42 ; 43 ; 45a) sont écrasés de telle sorte que la forme transversale des fils épais (42 ; 43 ; 45a) est formée pour avoir une forme sensiblement semi-circulaire, et
un diamètre interne (DI) et un diamètre externe (DE) du corps de bobine (30 ; 32) sont constants dans la partie dans laquelle les fils épais (42 ; 43 ; 45a) sont écrasés.

2. Cathéter (1 ; 3 ; 4) selon la revendication 1, dans lequel une portion droite de la forme sensiblement semi-circulaire des fils épais (42 ; 45a) est disposée sur un côté externe du corps de bobine (30).

3. Cathéter (2) selon la revendication 1, dans lequel une portion droite de la forme sensiblement semi-circulaire des fils épais (43) est disposée sur un côté interne du corps de bobine (32).

4. Cathéter (3 ; 4) selon l'une quelconque des revendications 1 à 3, dans lequel
le corps de bobine (34 ; 36) a une extrémité proximale et une extrémité distale,
la partie longueur dans laquelle les fils épais (45a) sont écrasés est au niveau d'une portion d'extrémité distale du corps de bobine (34 ; 36), et
le corps de bobine (34 ; 36) a en outre une partie longueur dans laquelle les fils épais (45b) ne sont pas écrasés, au niveau de sa portion d'extrémité proximale.

5. Cathéter (3 ; 4) selon la revendication 4, dans lequel les fils épais (45b) dépassent sur le côté externe du corps de bobine (34 ; 36) dans la partie longueur dans laquelle les fils épais (45b) ne sont pas écrasés.

6. Cathéter (4) selon l'une quelconque des revendications 1 à 5, dans lequel
une couche tressée (80) est disposée entre la couche interne (20) et le corps de bobine (36).
